(19) Europäisches Patentamt / European Patent Office / Office européen des brevets

(11) **EP 2 803 996 A1**

(12) **DEMANDE DE BREVET EUROPEEN**

(43) Date de publication:
**19.11.2014 Bulletin 2014/47**

(51) Int Cl.:
***G01N 33/18*** *(2006.01)*

(21) Numéro de dépôt: **13290107.5**

(22) Date de dépôt: **15.05.2013**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(71) Demandeur: **Merck Patent GmbH**
**64293 Darmstadt (DE)**

(72) Inventeurs:
• **Rajagopalan, Pascal**
**91120 Palaiseau (FR)**
• **Gaignet, Yves**
**78180 Montigny Le Bretonneux (FR)**
• **Glipa, Celine**
**78150 Rocquencourt (FR)**

(54) **Dispositif de mesure de conductivité d'un liquide pour déterminer de très bas niveaux de carbone organique total (TOC) dans de l'eau pure et ultra-pure**

(57) La présente demande concerne un dispositif (1) de mesure de conductivité d'un liquide qui comprend une chambre de mesure pour contenir un volume d'échantillonnage à irradier par rayons UV formée dans un corps hydraulique (4) qui comprend un canal d'entrée pour alimenter la chambre de mesure en liquide à mesurer et un canal de sortie pour évacuer le liquide mesuré de la chambre de mesure, le canal d'entrée et le canal de sortie débouchant de part et d'autre hors d'une surface exposée aux rayons UV, de sorte que seul le volume d'échantillonnage contenu dans la chambre de mesure est irradié. La présente demande vise aussi une utilisation d'un tel dispositif ainsi qu'un système de purification comprenant un tel dispositif.

Fig.2

## Description

La présente invention concerne, de manière générale, un dispositif de mesure de conductivité d'un liquide ou un fluide pur voire ultra-pur, notamment de l'eau ultra-pure, en particulier pour des dispositifs de mesure de substances organiques ou de carbone organique total (« *Total Organic Carbon* » ou « *TOC* » en anglais) dans un échantillon de liquide ou fluide.

Elle vise plus particulièrement un tel dispositif pour des mesures à de très faibles niveaux d'impuretés (typiquement moins de 500 voire 5 ppb, « parts per billion » en anglais, soit « partie par milliard » en français) dans un fluide ou un liquide ultra-pur, tel une eau ultra-pure, i.e. dont la conductivité est en pratique inférieure à 0,055 μS/cm (c'est-à-dire moins de 0,055 micro-siemens par centimètre).

Elle vise aussi un matériau pour la réalisation d'au moins une partie d'une cellule de mesure.

De nombreuses applications technologiques modernes ont besoin, pour leur fonctionnement, d'une eau ultra-pure, en particulier dans les industries chimiques, pharmaceutiques, médicales et électroniques.

Dans l'eau théoriquement ultra-pure, les deux seules espèces ioniques présentes proviennent de la dissociation des molécules d'eau en $H^+$ et $OH^-$.

Ainsi, à 25°, la conductivité théorique d'un échantillon d'eau exempt de contaminants ioniques est égale à 0,055 μS/cm, c'est-à-dire une résistivité (inverse de la conductivité) égale à 18,2 MΩ.cm.

Cette conductivité est mesurée en appliquant un potentiel électrique entre deux électrodes de mesure plongées dans l'échantillon d'eau. Elle est déterminée à partir de la tension et de l'intensité du courant produit à l'intérieur d'une chambre de mesure de conductivité.

On rappelle, à cet égard, que la conductivité est la mesure du flux d'électrons qui traversent une substance. Elle est directement proportionnelle à la concentration en ions, à la charge portée par chacun de ces ions (valence) et à leur mobilité. Cette mobilité est fonction de la température, et par conséquent, la mesure de la conductivité dépend elle aussi de la température.

En effet, l'un des problèmes majeurs dans le domaine de la mesure de conductivité est que celle-ci est grandement affectée par les variations de température : plus la température d'un échantillon est élevée, plus la résistivité est moindre (à cause de la mobilité des ions). Ainsi, pour assurer une mesure précise, il est nécessaire de compenser la mesure de la conductivité en température. A cet effet, les cellules de mesure de conductivité sont généralement équipées de capteurs de température de l'échantillon.

Un capteur de température, se présentant en pratique sous la forme d'une thermistance, est généralement prévu pour être placé soit en amont, soit en aval de la cellule de mesure de conductivité ou, mieux encore, positionné sous une des électrodes avec, éventuellement, une fine interface de verre entre le capteur de température et une chambre recevant l'échantillon à mesurer.

La calibration dans un tel dispositif est donc facilitée par l'intégration d'une thermistance menant à une meilleure précision de mesure de température et conductivité.

Cette mesure de conductivité est aussi affectée par la géométrie de la chambre recevant l'échantillon.

Ceci est particulièrement important quand il s'agit d'eau ultra-pure.

En pratique, un échantillon d'eau théoriquement ultra-pure est soumis à une photo-oxydation au moyen de rayons ultraviolets (UV), ce qui permet de mesurer la quantité de carbone organique présente dans l'eau à partir de la baisse de résistivité résultant de l'oxydation aux ultraviolets des substances organiques présentes dans l'échantillon d'eau soumis à la mesure.

Or, il est préférable que la surface (S) de matériaux exposés aux rayons UV soit la plus faible possible pour minimiser un lessivage des matériaux durant l'écoulement de fluide dans la chambre (moins il y a de polluants, meilleure est la mesure), mais tout en conservant un volume (V) d'échantillonnage le plus important possible pour avoir un meilleur rendement et une meilleure efficacité. Autrement dit, il est préférable d'avoir un ratio (S/V) le plus petit possible. Parallèlement, plus le volume d'échantillon est petit, plus un temps nécessaire à la mesure de conductivité (mesure directe) de l'eau ultra-pure ou de tout autre liquide ultra-pur à mesurer est court.

On connait par exemple le document EP 1 927 849 qui décrit un dispositif de mesure de conductivité comprenant une cellule de mesure composée d'une fenêtre transparente aux rayons ultraviolets (UV) et pourvue d'une chambre de mesure destinée à recevoir l'échantillon de liquide à mesurer, et d'un substrat comprenant des électrodes formant un fond de la chambre. Le substrat présente ainsi une double fonction, à savoir une fonction de mesure (du fait qu'il comprend des électrodes) et une fonction hydraulique car il comprend des trous permettant d'alimenter et vider la chambre de mesure. Une telle conception du substrat induit un coût de revient élevé ainsi qu'une résistance mécanique affaiblie (surtout quand le substrat présente initialement une épaisseur faible) de par la présence des trous ayant dû y être percés. La présence des trous implique aussi des dimensions minimum à respecter en termes de longueur et largeur pour concevoir la chambre. La conception de la fenêtre comprenant la chambre, de façon monobloc, est également difficile, compliquée et coûteuse. Par ailleurs, dans une telle conception, les trous d'arrivée et de sortie du fluide dans la chambre se retrouvent en vis-à-vis des rayons UV qui naturellement pénètrent à l'intérieur. Le volume d'échantillonnage est par conséquent difficile à déterminer du fait de l'incertitude portant sur les volumes irradiés par ces trous.

On connait aussi par exemple le document US 6 444 474 qui décrit un dispositif de mesure de conductivité permettant une oxydation rapide des composés organiques présents dans le fluide contenant jusqu'à 100 ppm (on entend ici par rapide que la réaction dure de l'ordre de 2 à 30 secondes d'après ce document), ainsi qu'un rinçage du capteur. Selon certains des modes de réalisation, le dispositif comprend une cellule formée en trois parties indépendantes, à savoir un substrat supérieur transparent aux UV et comprenant une électrode, un substrat inférieur comprenant une autre électrode et un écarteur comprenant une cavité, de sorte que la chambre est ainsi formée par assemblage des trois éléments. La chambre présente un faible volume (V) (moins de 30 μL) et une épaisseur inférieure à 150 μm. Le dispositif utilise deux électrodes spécifiques permettant d'obtenir une photo-catalyse pendant l'irradiation aux UV et est en outre composé de matériaux tels qu'ils constituent des sources de pollution du fluide (phénomène de lessivage), ce qui accroît la conductivité du fluide et rend ainsi impossible de réaliser des mesures à des niveaux de ppb très faibles. De par ses dimensions, la chambre du dispositif décrit présente ici un ratio entre la surface des matériaux irradiés (S) et son volume (V) de l'ordre de 14 mm$^2$/μL. Enfin, des trous sont aussi formés dans le substrat inférieur pour alimenter et vider la chambre de fluide, engendrant, entre autres, au moins certains des inconvénients précités.

Par conséquent, la présente invention, conçue plus spécifiquement pour l'analyse de TOC inférieur à 500 ppb sur des eaux de conductivité entre 0,055 et 1 μS/cm à 25°C (c'est-à-dire pures voire ultra-pures), a, de manière générale, pour objet un dispositif permettant de résoudre au moins en partie les inconvénients précités, et conduisant en outre à d'autres avantages.

De manière plus précise, elle a tout d'abord pour objet un dispositif de mesure de conductivité d'un liquide, de type eaux pures ou ultra-pures (présentant pas exemple une conductivité inférieure à 1μS/cm et moins de 500ppb), comprenant une chambre de mesure pour contenir un volume d'échantillonnage à irradier par rayons UV, une fenêtre transparente aux UV étant située entre la chambre de mesure et une source de rayons UV en fermant hermétiquement un premier côté de la chambre de mesure, caractérisé en ce que la chambre est formée dans un corps hydraulique, la chambre de mesure débouchant au moins du premier côté sur une première surface du corps hydraulique, la fenêtre transparente aux UV recouvrant au moins une partie de la première surface en fermant la chambre de mesure hermétiquement du côté de la première surface, et en ce que le corps hydraulique comprend un canal d'entrée pour alimenter la chambre de mesure en liquide à mesurer et un canal de sortie pour évacuer le liquide mesuré de la chambre de mesure, le canal d'entrée et le canal de sortie débouchant de part et d'autre hors d'une surface exposée aux rayons UV, de sorte que seul le volume d'échantillonnage contenu dans la chambre de mesure est irradié.

Un tel dispositif permet de mesurer de très basses teneurs en TOC (typiquement en-dessous de 500 ppb et de préférence 5 ppb), d'éléments oxydables transformés en $CO_2$ (dioxyde de carbone), par irradiation par une source de rayons UV (ultraviolets), de longueur d'onde typiquement comprise entre 160nm et 400 nm (nanomètres), dans une eau pure et même dans une eau ultra-pure (c'est-à-dire respectivement à 1μS/cm et 0,055μS/cm).

Un tel positionnement des bouches d'arrivée et de sortie de l'eau dans la chambre permet de réduire une surface active du fond de la chambre (comprenant généralement un capteur), et de mieux pouvoir déterminer le volume d'échantillonnage contenu dans la chambre puisque les bouches (des trous) sont désormais hors du vis-à-vis des rayons UV.

Pour réduire encore la surface de la chambre, le dispositif est par exemple en outre dépourvu d'électrodes de photo-catalyse.

Selon un exemple de réalisation, le corps hydraulique comprend une entrée à laquelle est relié d'une part le canal d'entrée, et une sortie à laquelle est relié d'une part le canal de sortie, le canal d'entrée et le canal de sortie débouchant d'autre part dans la chambre par une paroi latérale délimitant latéralement la chambre de mesure.

Ainsi, les rayons UV étant en vis-à-vis du fond de la chambre, les rayons ne peuvent plus pénétrer dans les profondeurs des canaux d'entrée et de sortie. En outre, les rayons UV sont généralement émis de façon parallèle à la paroi latérale de la chambre, celle-ci étant par exemple de forme globalement parallélépipédique.

Selon un exemple de réalisation, la chambre de mesure est fermée d'un côté d'une deuxième surface du corps hydraulique par un fond faisant partie intégrante du corps hydraulique.

Selon un exemple de réalisation, la chambre de mesure est fermée hermétiquement d'un côté d'une deuxième surface du corps hydraulique par un substrat comportant au moins deux électrodes de mesure de conductivité, le substrat étant appliqué contre au moins une partie de la deuxième surface du corps hydraulique tel que les électrodes sont en vis-à-vis de la chambre de mesure.

Et par exemple, l'entrée et la sortie sont formées dans le corps hydraulique hors d'une surface de contact définie entre le substrat et la deuxième surface.

Une telle disposition permet notamment d'éviter d'avoir à réaliser un trou dans le substrat, de l'usiner, ce qui le fragilise mécaniquement.

Ainsi, le substrat, supportant les électrodes formant le capteur qui sont ici réalisées par exemple par un circuit imprimé sur le substrat, est indépendant du circuit hydraulique, et en particulier du corps hydraulique comprenant les éléments mécaniques relatifs à l'écoulement de fluide ; il est donc par exemple inutile de percer le substrat.

De plus, le corps hydraulique dissocie aussi les fonctions associées à la présence d'une fenêtre transparente aux UV, ce qui permet d'utiliser un matériau non transmissif de ce point de vue pour la réalisation du corps hydraulique.

Cette indépendance du corps hydraulique pour former la chambre permet aussi de disposer de plus grandes latitudes de conception, comme cela est détaillé plus loin dans cet exposé.

Pour améliorer l'écoulement, notamment en minimisant des apparitions potentielles de bulles, par exemple, le canal d'entrée et le canal de sortie comprennent chacun au moins une portion débouchant dans la chambre parallèlement à la fenêtre.

Afin d'améliorer aussi le rinçage du dispositif, et notamment de la chambre de mesure, la forme de la chambre et la circulation du fluide sont à concevoir de sorte à éviter des zones résiduaires, où le fluide pourrait stagner, être bloqué, induisant par exemple des pertes de charges. La circulation du fluide est très importante, non seulement pour éviter que des bulles soient piégées pendant les réactions d'oxydation, mais aussi pour faciliter le rinçage de la cellule avant oxydation.

Pour remédier au moins en partie à ces inconvénients, une entrée et une sortie dans la chambre sont parallèles voire tangentes au capteur (les électrodes) facilitant l'élimination des bulles et favorisant le rinçage, et de préférence, lors de son utilisation, la cellule est positionnée verticalement, c'est-à-dire de manière telle que l'entrée de fluide dans la chambre soit en positionnée en bas et la sortie du fluide de la chambre soit positionnée en haut.

A défaut, il est préférable que les électrodes soient situées sous la fenêtre pour s'assurer que les électrodes sont bien immergées dans l'échantillon pour réaliser les mesures.

Selon certaines dispositions particulières, une portion du canal d'entrée et une portion du canal de sortie débouchent dans la chambre parallèlement à la fenêtre et sont formées chacune dans le corps hydraulique par une gorge creusée dans la partie de la première surface recouverte par la fenêtre transparente aux UV.

Ceci permet par exemple une réalisation plus simple du corps hydraulique, notamment pour la réalisation des conduits, et lors de son utilisation de pouvoir nettoyer le circuit hydraulique, notamment les conduits, plus facilement.

De plus, selon un mode de conception particulier du dispositif, toute zone favorisant des pertes de charges pendant le rinçage est évitée, et pour cela, par exemple, au moins l'un du canal d'entrée et du canal de sortie présente un coude arrondi. L'existence d'un angle aigu et/ou d'une zone à faible de débit est alors évitée. Une telle configuration permet de minimiser les turbulences dans l'écoulement, et le rendre le plus laminaire possible, notamment lorsque les canaux comprennent une déviation. Ceci peut aussi être favorisé, indépendamment ou en combinaison, par des coins arrondis ou chanfreinés de la paroi latérale de la chambre de mesure qui présente par exemple globalement une forme rectangulaire.

Selon un mode de réalisation de l'invention, la deuxième surface du corps hydraulique comprend une empreinte délimitée par un contour entourant la chambre pour positionner le substrat comprenant des électrodes de sorte que les électrodes sont en vis-à-vis de la chambre de mesure, et l'empreinte étant creusée dans le corps hydraulique et de taille ajustée au substrat pour l'y loger.

Selon un autre mode de réalisation de l'invention, la première surface du corps hydraulique comprend une empreinte délimitée par un contour entourant la chambre de mesure, pour positionner la fenêtre transparente aux UV, et l'empreinte étant creusée dans le corps hydraulique et de taille ajustée à la fenêtre pour l'y loger.

De plus, il est intéressant que le substrat supporte les fonctions de mesure et de connexion électrique, par exemple grâce à un ruban électrique, également désigné ici « FPC » de l'anglais « Flexible Printed Circuit board » brasé dessus. L'ensemble de l'assemblage est simplifié d'un point de vue mécanique. A cet effet, selon un mode de réalisation, la deuxième surface du corps hydraulique comprend une creusure pour faire passer un FPC brasé sur le substrat.

Le dispositif comporte, en outre, par exemple, un boitier en deux parties à la faveur duquel est logée la cellule (c'est-à-dire l'ensemble formé de la fenêtre, du corps hydraulique et du substrat), et des moyens d'assemblage par serrage des deux parties, propres à assurer l'étanchéité de la cellule de mesure de conductivité.

Une partie supérieure forme par exemple un support permettant de maintenir une lampe à UV et une partie inférieure qui forme par exemple un socle. Le support de la lampe UV, c'est-à-dire la partie supérieure du boitier, permet par exemple un assemblage de l'ensemble des composants mécaniques plus rapide et plus facile, et permet d'établir un alignement précis de ces éléments.

Le corps hydraulique permet aussi de protéger des joints toriques, présents par exemple autour de l'entrée et de la sortie de fluide du corps hydraulique, et d'autres éléments. En effet, par exemple dans le dispositif décrit dans le document EP 1 927 849 précédemment cité, les joints toriques étaient directement exposés aux rayons UV, ce qui provoquait un vieillissement rapide, voire prématurés des éléments irradiés.

De plus, grâce à la présente invention, le volume d'échantillon est facilement reproductible. Par ailleurs, les volumes réalisables étaient auparavant liés à la taille du substrat qui comprenait deux orifices pour faire circuler le fluide dans la chambre. La présence de ces orifices dans le substrat, dont la surface venait s'additionner à celles des capteurs de conductivité et de température, constituait ainsi un facteur limitant pour réaliser des chambres de petites dimensions.

L'assemblage des éléments de la cellule comprend aussi par exemple des joints pour améliorer l'étanchéité de l'ensemble. Une faible valeur de pression, procurée par le serrage entre les deux parties du boitier, est alors suffisante pour retenir les éléments les unes contre les autres afin d'assurer l'étanchéité. Ainsi, il n'y a pas besoin de coller les éléments ensemble, ce qui permet notamment d'éliminer les contaminants organiques provenant d'une colle et entraînant des erreurs de mesure dans le cas de la mesure de TOC.

Et par exemple, le dispositif comprend un moyen de mesure de la température dans la chambre de mesure. Le moyen de mesure de la température est par exemple une thermistance.

Selon un exemple de réalisation, la thermistance est logée au sein d'une électrode de mesure.

Selon un mode de réalisation sans substrat, l'électrode de mesure comprenant la thermistance est introduite dans la chambre de mesure via un orifice formé latéralement dans le corps hydraulique. Ainsi, l'électrode est positionnée à travers le courant de fluide, elle croise l'écoulement, si possible orthogonalement pour mieux assurer son immersion dans l'échantillon.

En ce qui concerne les sources de contamination, des particules provenant des différents matériaux constitutifs de la cellule et en contact avec le fluide à mesurer sont extrêmement critiques pour les mesures de très basses teneurs (i.e. de 1 à 10 ppb par exemple).

Des études et essais ont été réalisés sur des corps hydrauliques réalisés en différents matériaux et sont récapitulés dans le tableau suivant :

| Matériau constitutif du corps | Lessivage du fond par de l'eau ultra-pure | | Procédé de fabrication |
|---|---|---|---|
| | ppb/min | µS/cm/min | |
| Silicone | 200 | 0,988 | |
| PTFE (Teflon) | 5 | 0,1187 | Moulage par injection |
| Alumine anodisée | 0,9 | 0,064 | Usinage |
| Inox 316 | 1,3 | 0,069 | Usinage |
| Céramique usinable (MACOR®) | <0,3 | <0,058 | Usinage |
| Céramique (avec 1 % de liant éliminé au cour du procédé de cuisson) | 0,6 | 0,061 | Moulage par injection |

Tous les pourcentages relatifs aux compositions sont ici des pourcentages massiques.

Il en a résulté qu'un corps hydraulique en céramique, et en particulier en céramique à base d'oxyde d'aluminium, s'est révélé être particulièrement performant pour réduire notablement la teneur de contaminant dans le fluide.

De préférence, le corps hydraulique est en céramique comprenant au moins 16 % d'alumine.

Par exemple, le corps hydraulique est en céramique (avec 1% de liant éliminé au cours du procédé de cuisson de la céramique), réalisé par exemple par moulage par injection, green-machining ou usinage. Soumis à un lessivage par de l'eau ultra-pure, il a produit une pollution de 0,6 ppb/min et une variation de conductivité de 0,061 µS/cm/min.

Et par exemple, la céramique du corps hydraulique est une vitrocéramique usinable.

Selon un exemple de céramique obtenue par green-machining, le corps hydraulique est composé d'une céramique comprenant au moins 99% d'alumine, voire 100% d'alumine, à quelques impuretés près. Un tel matériau étant par exemple obtenu par green-machining, la matière première comprend alors un liant (environ 1 wt. %) consommé pendant le procédé de cuisson (après injection dans un moule).

Le green-machining s'applique typiquement à la céramique avant cuisson (« unfired state » selon la terminologie anglo-saxonne). A cette étape le matériau est encore relativement mou (« chalky » selon la terminologie anglo-saxonne) et peut contenir certains additifs.

Selon un mode de réalisation particulièrement intéressant, s'est avéré particulièrement performant un corps hydraulique en céramique usinable, présentant par exemple une composition du type :

- 46% de dioxyde de silice ($SiO_2$)
- 17% de magnésie ($MgO$)
- 16% d'alumine ($Al_2O_3$)
- 10% d'oxyde de potassium ($K_2O$)
- 7% d'oxyde de bore ($B_2O_3$)
- 4% de fluor (F)

Il a, quant à lui, produit une pollution inférieure à 0,3 ppb/min et une variation de conductivité inférieure à 0,058

μS/cm/min.

Un exemple de céramique usinable de ce type est le MACOR®.

L'utilisation de tels matériaux permet ainsi une bien meilleure tenue au lessivage induit par les écoulements de fluide.

Ainsi, selon un mode de réalisation envisageable, le corps hydraulique est en céramique moulée par injection. Ou, selon un autre mode de réalisation privilégié, le corps hydraulique est en MACOR®.

Quelle que soit la configuration de la cellule, il est alors particulièrement avantageux de réaliser le corps hydraulique en céramique à au moins 16% d'alumine, et de préférence en MACOR® afin de réduire au mieux les sources de contamination possible.

Il s'agit donc d'un aspect original en soi de la présente invention.

Ainsi, selon un autre aspect, est proposé un dispositif de mesure de conductivité d'un liquide, comprenant une fenêtre transparente aux UV, et une chambre de mesure formée au moins en partie dans un écarteur, l'écarteur étant réalisé en céramique comprenant au moins 16% d'alumine, par exemple en céramique moulée par injection ou usinable, et de préférence en MACOR®.

Selon un exemple de réalisation, l'écarteur est un corps hydraulique tel que décrit précédemment, ou un écarteur tel que décrit dans le document US 6 444 474.

Selon un autre aspect, la chambre peut également être constituée d'un métal noble (titane (Ti), or (Au), ou encore platine (Pt) par exemple). Toutefois, pour des questions de coût de matière, il est difficilement envisageable industriellement de réaliser un corps hydraulique en métal noble massif. Une métallisation à partir d'une pièce usinée dans un métal plus standard, comme par exemple en acier inoxydable, est en revanche extrêmement plus économique. Il s'agit par exemple d'un procédé de métallisation sous vide (« sputtering » en anglais). Ainsi il est possible de faire usiner une pièce, par exemple en acier inoxydable, puis de revêtir sa surface des matériaux suivants au choix, donnés à titre d'exemples :

- métaux nobles : Pt, Ti, Au ;
- oxydes de métaux nobles : $TiO_2$ ;
- ou de leurs alliages : $TiO_2$-Pt...

Les dépôts sont par exemple réalisés par pulvérisation cathodique magnétron. La pièce à revêtir est optionnellement animée d'un mouvement de rotation planétaire pour optimiser l'uniformité du revêtement.

En outre, préalablement au dépôt, une phase d'activation par plasma peut être mise en oeuvre pour améliorer l'adhérence du revêtement.

Ce même procédé de métallisation sous vide peut aussi être réalisé sur les différents joints en contact avec l'eau. Le joint conserve alors ses propriétés d'élasticité et de compression. Un réseau de fissure métallique se forme ainsi à la surface du joint lorsqu'il est étiré. Ainsi le niveau de relargage lors de l'irradiation UV peut être encore diminué dans la chambre.

Par ailleurs, comme mentionné précédemment, dans le cas de l'application considérée, la présente invention permet d'optimiser la chambre de mesure de TOC sur le plan du volume et de l'épaisseur de la couche de liquide afin que ceux-ci permettent de garantir une photo-oxydation aux UV efficace dans un temps raisonnable pour éviter les dérives de la mesure dues aux variation importantes de la température de l'échantillon et aux extractions de matière organiques provenant des matériaux pendant le temps de photo-oxydation.

Indépendamment du matériau, un autre paramètre permettant de réduire l'oxydation des matériaux constituant la cellule est de minimiser le ratio (SN) entre la surface (S) des matériaux exposés aux UV et le volume (V) de l'échantillon mesuré.

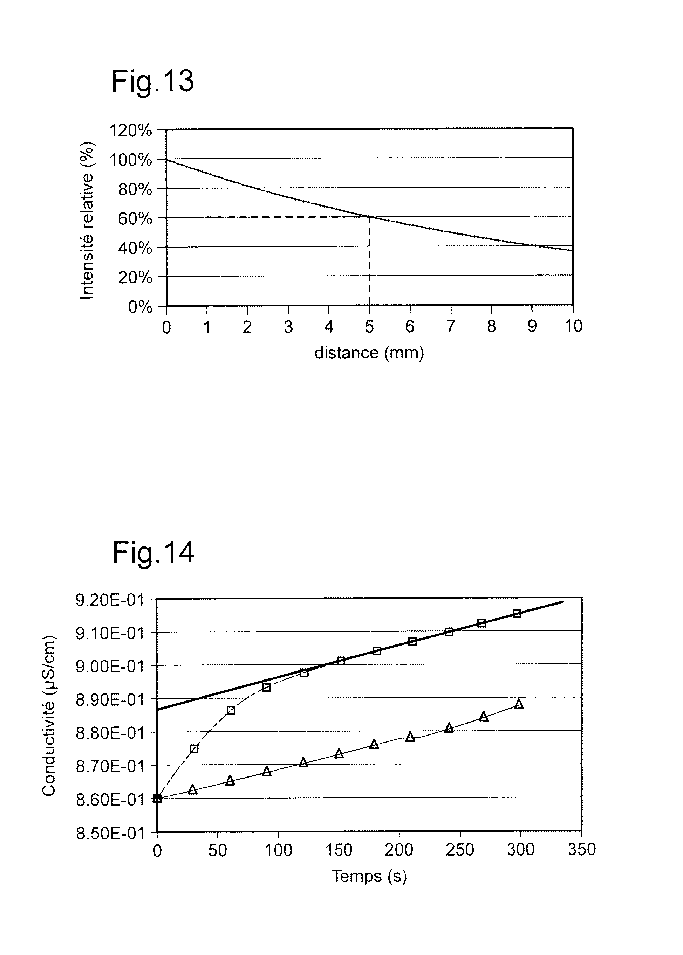

Comme expliqué précédemment, il est préférable de minimiser la surface de matériaux exposés pour minimiser le lessivage. En développant un dispositif tel que décrit précédemment, il est possible de limiter la surface active de la chambre comprenant le capteur (constitué par exemple d'un substrat, optionnellement avec un FPC, ou d'électrodes individuelles - cette surface correspond généralement à la surface du fond de la chambre de mesure), de préférence dépourvu d'électrodes de photo-catalyse, en plaçant les bouches d'entrée et de sortie du fluide parallèlement au capteur (et/ou à la fenêtre) c'est-à-dire plus généralement hors de la surface active, du fond, et, maximiser le volume d'échantillonnage en fonction de l'épaisseur du fluide (cette épaisseur étant limitée par l'absorption des rayons UV comme décrit ultérieurement en référence à la figure 13).

Ainsi, il est possible de miniaturiser la cellule tout en gardant cette limite de ratio (SN) inférieur (ou égal) à 2 $mm^2/\mu L$ et tout en gardant une épaisseur de liquide facilement photo-oxydable et un volume raisonnable, et ainsi pouvoir mesurer des faibles valeurs de TOC (inférieur à 5 ppb) dans un temps de photo-oxydation inférieur à trois minutes.

Un dispositif tel que décrit précédemment permet une plus grande latitude de conception. En effet, du fait que dans les dispositifs connus, le substrat était percé, il existait des dimensions du substrat, et par conséquent de la chambre, en-deçà de quoi le substrat devenait trop fragile, voire très difficile ou impossible à réaliser. Eviter de percer le substrat permet ainsi de réaliser des cellules d'échelle bien moindre.

Ainsi, selon un mode de réalisation de l'invention, la surface exposée est au total de 430 mm$^2$ pour un volume d'échantillonnage de 390 µL. Le ratio entre la surface du matériau et le volume d'échantillonnage est ainsi de 1,1 mm$^2$/µL, contre au moins 13,6 mm$^2$/µL pour des chambres selon le document US 6 444 474. Une cellule selon un mode de réalisation de la présente invention permet alors de réduire d'un facteur dix la teneur en particules résiduelles dues au lessivage.

La chambre de mesure d'un dispositif, selon un mode particulièrement intéressant de réalisation de l'invention, présente donc un ratio (SN) inférieur ou égal à 2mm$^2$/µL, où (S) est la surface de matériau irradié, et (V) est le volume d'échantillon de fluide, et par exemple de 1,1 mm$^2$/µL. De telles valeurs n'étaient pas atteignables auparavant à cause des contraintes liées aux conceptions des dispositifs de l'art antérieur.

De tels ratios sont par exemple obtenus avec une hauteur de chambre inférieure à 4 mm afin que l'ensemble de l'échantillon contenu reçoive au moins 60% des rayons UV, i.e. pour conserver assez de puissance d'irradiation, et ce, tout en minimisant la surface, ce qui est possible grâce à une absence de trous et d'électrodes de photo-catalyse.

Par exemple, la chambre de mesure présente une épaisseur (e) comprise entre 0,5 mm et 4 mm.

Selon un exemple de réalisation, la chambre de mesure présente un volume (V) supérieur ou égal à 100 µL, voire de préférence supérieur ou égal à 400 µL.

Selon un exemple de réalisation, la chambre de mesure présente une surface de matériau irradié (S) inférieure ou égale à 600 mm$^2$.

Une faible épaisseur de liquide à mesurer maximise l'irradiation par rayons UV et le positionnement des capteurs (des électrodes de mesure principalement) n'occulte pas les radiations. Ceci permet une oxydation rapide et complète des composés organiques.

Les durées de rinçage et de mesure sont réduites et peuvent être facilement synchronisées avec un dispositif de référence.

Enfin, la qualité de mesure de conductivité correctement compensée en température pendant la calibration pour différentes teneurs en particules organiques permet une meilleure calibration et une plus grande précision de la valeur de TOC associée mesurée ultérieurement.

Un dispositif selon l'invention permet ainsi de réduire les résidus de lessivage jusqu'à atteindre une variation de conductivité de 0,0055 µS/cm/min, ce qui signifie en d'autres termes jusqu'à mille fois moins de polluants que dans des dispositifs de l'art antérieur dans des conditions identiques.

La présente invention vise aussi une utilisation d'un dispositif tel que décrit précédemment, dans laquelle le dispositif est positionné de sorte qu'un écoulement de fluide dans la chambre de mesure soit vertical et ascendant, le canal d'entrée débouchant dans la chambre sous le canal de sortie. Le montage vertical de la cellule avec la sortie vers le haut permet de faciliter le rinçage et le dégazage.

Et enfin, la présente invention vise un système de purification d'eau, comprenant un dispositif tel que décrit précédemment, le dispositif étant fixé sur une carte électronique tel que le canal d'entrée débouche dans la chambre sous le canal de sortie de sorte qu'un écoulement de fluide dans la chambre de mesure soit vertical et ascendant, le canal d'entrée et le canal de sortie présentant au moins chacun une portion dans le prolongement l'une de l'autre, les portions débouchant dans la chambre de mesure en vis-à-vis.

Un tel système est par exemple un système de production et de purification d'eau (comme par exemple le produit commercial Milli-Q (marque déposée)) ou un système de boucle de distribution d'eau purifiée.

Par exemple, il présente en outre les avantages suivants :

- Il permet de détecter de faibles teneurs en TOC dans de l'eau pure ou ultra-pure, et avec une meilleure précision,
- Il présente une moins grande dispersion de mesure,
- Les valeurs de TOC mesurée sont plus proches des références microélectroniques que d'autres dispositifs
- Il a un faible cout de conception (ne nécessite pas d'usinage de cavité ou d'extrusion),
- Une telle cellule est compacte,
- La phase de rinçage est plus rapide,
- La phase d'oxydation est plus rapide.

L'invention sera bien comprise et ses avantages apparaitront mieux à la lecture de la description détaillée qui suit, en référence aux dessins annexés (dont les échelles ne sont pas représentatives) donnés à titre illustratif et nullement limitatif, sur lesquels :

La figure 1 représente différentes vues d'un dispositif selon un mode de réalisation de l'invention,
La figure 2 représente une vue éclatée d'un dispositif selon un mode de réalisation de l'invention,
La figure 3 présente une vue en transparence d'un socle d'un dispositif selon un exemple de réalisation,
La figure 4 illustre un corps hydraulique vu de dessus selon un premier exemple de réalisation de la présente invention,

La figure 5 illustre le corps hydraulique de la figure 4 vu de dessous,

La figure 6 montre une vue de dessus d'un assemblage d'un corps hydraulique selon les figures 4 et 5, d'un substrat comprenant des électrodes imprimées et d'un socle selon un exemple de réalisation.

La figure 7 présente un corps hydraulique vu de dessus selon un deuxième exemple de réalisation de la présente invention,

La figure 8 présente le corps hydraulique de la figure 7 vu de dessous,

La figure 9 présente un corps hydraulique vu de dessus selon un troisième exemple de réalisation de la présente invention,

La figure 10 détaille une électrode comprenant une thermistance pouvant par exemple être insérée dans le corps hydraulique de la figure 9,

La figure 11 présente un exemple de réalisation d'un substrat surmoulé sur deux électrodes telles que présentées figure 10,

La figure 12 est un tableau regroupant des dimensions et des caractéristiques de chambres de mesure décrites dans l'US 6 444 474 et selon des exemples de modes de réalisation de la présente invention,

La figure 13 permet de constater l'absorption d'un rayonnement dans une couche d'eau en fonction de la profondeur,

La figure 14 présente des courbes d'extraction en fonction du temps permettant d'apprécier les performances d'un dispositif selon un exemple de réalisation de la présente invention.

Il convient de relever à cet égard que la description qui suit est celle de modes de réalisation préférés, donnés à titre d'exemples non limitatifs.

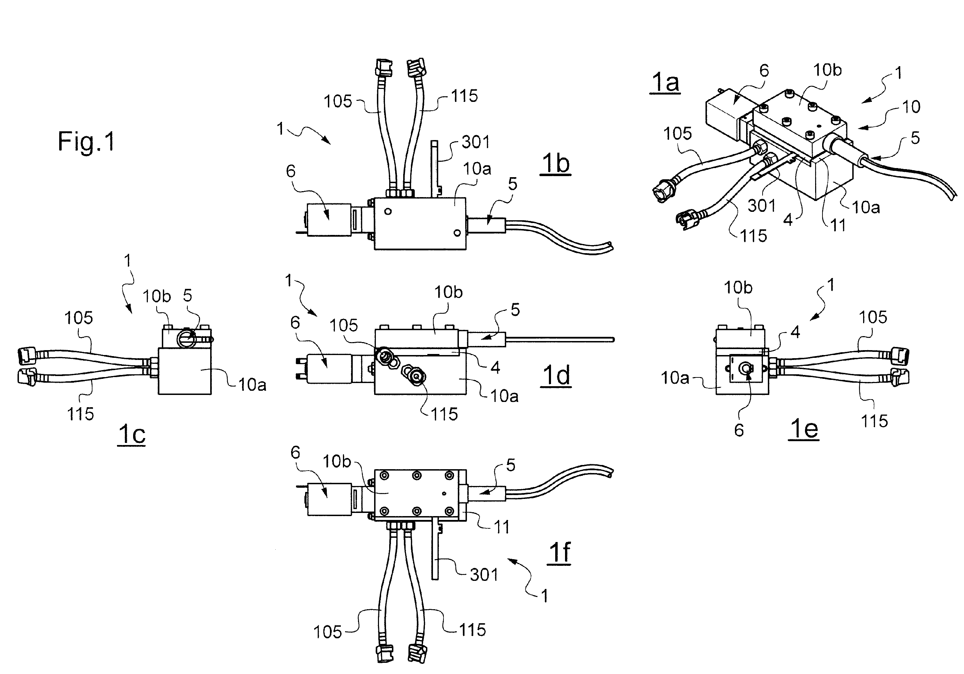

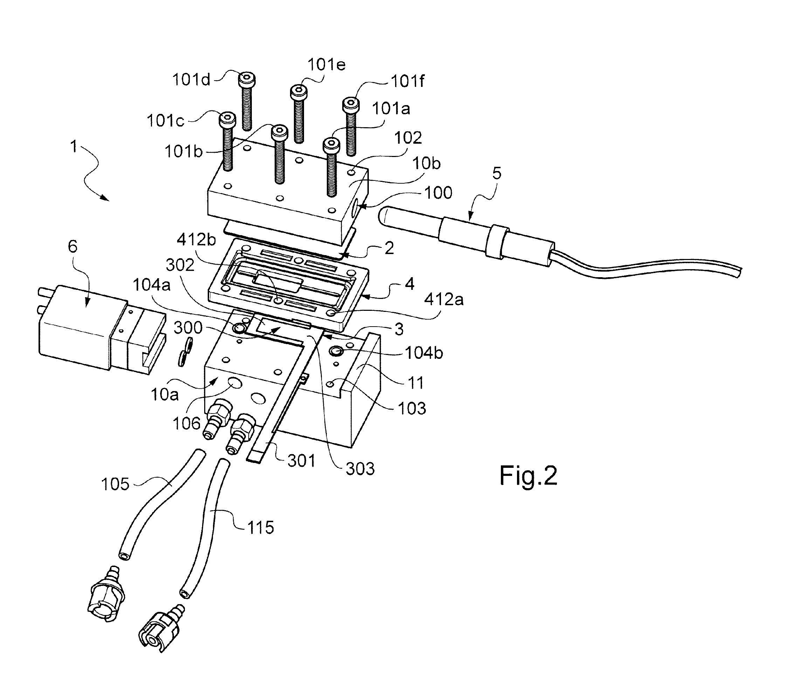

En référence aux figures 1 et 2, un dispositif de mesure de conductivité 1 d'un liquide ultra-pur, par exemple de l'eau ultra-pure, comporte un boîtier 10 en deux parties 10a, 10b. Une partie inférieure du boitier 10 du dispositif 1 constitue un socle 10a auquel est relié une électrovanne 6, et une partie supérieure du boitier 10 constitue principalement un support 10b d'une source de rayons UV, en l'occurrence une lampe à rayons UV 5, grâce à un logement 100 (visible figure 2), présent dans le support 10b, et prévu pour y loger la lampe 5.

Entre les deux parties 10a, 10b, sont logés une fenêtre 2 transparente aux UV et un corps hydraulique 4 comprenant une chambre de mesure 400 avec un fond qui a ici une forme globalement parallélépipédique rectangle. Ainsi, la fenêtre 2 et la chambre 400 avec son fond constituent ici la cellule de mesure de conductivité. Il est décrit ultérieurement que le fond peut être par exemple un substrat 3 ou une partie intégrante 421 du corps hydraulique 4, ou même une plaque indépendante (non représentée).

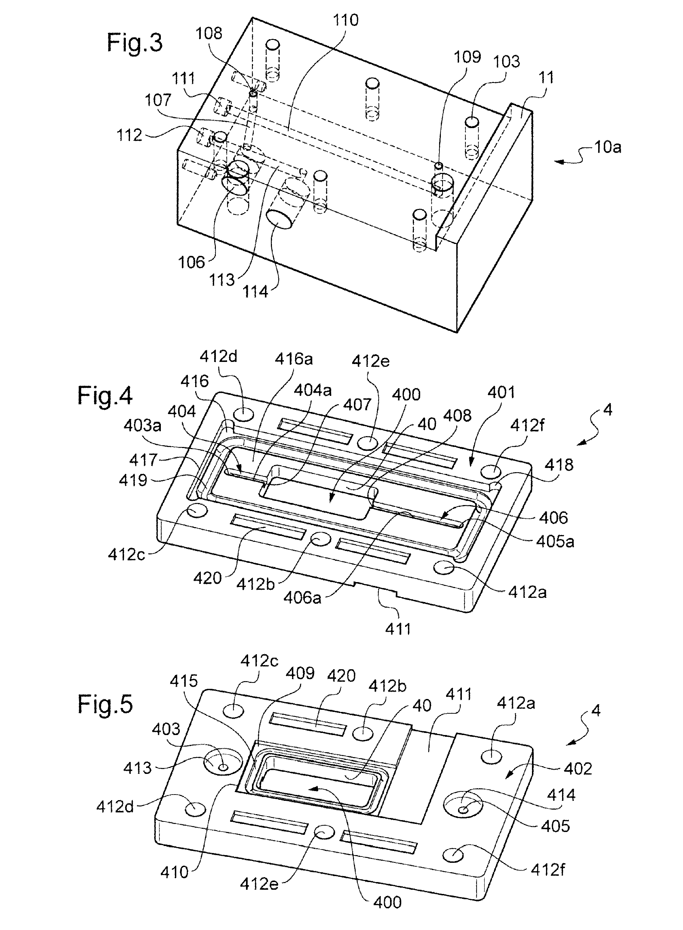

Pour réaliser une mesure, le liquide à analyser est amené via un flexible d'arrivée de liquide 105. Celui-ci est raccordé d'une part à un circuit hydraulique d'un système de purification de l'eau à analyser (non représenté), et d'autre part à une entrée 106 du socle 10a. Comme le montre la figure 3, le socle 10a comprend un premier conduit 107 reliant l'entrée 106 à un premier orifice 108, qui est positionné en vis-à-vis d'une entrée 403 du corps hydraulique 4 (visible par exemple figure 5) lorsque le dispositif 1 est assemblé.

Pour l'évacuation du fluide, le socle 10a du dispositif 1 présente un deuxième orifice 109 (voir figure 3), qui est positionné en vis-à-vis d'une sortie 405 du corps hydraulique 4 (voir figure 5) lorsque le dispositif 1 est assemblé. Un deuxième conduit 110 relie le deuxième orifice 109 à une entrée d'électrovanne 111 à laquelle est reliée l'électrovanne 6 d'une part. Après passage dans l'électrovanne 6, reliée d'autre part à une sortie d'électrovanne 112 formée dans le socle 10a, un troisième conduit 113 relie la sortie d'électrovanne 112 à une sortie 114 du dispositif 1. Un flexible de sortie de liquide 115 est ainsi raccordé d'une part à un réservoir de liquide ultra-pur mesuré (non représenté), et d'autre part à la sortie 114 du socle 10a.

Le liquide transite ainsi par le flexible d'arrivée 105, le premier conduit 107 puis un canal d'entrée 404 du corps hydraulique 4 avant d'arriver dans la chambre 400 où est réalisée la mesure.

Une fois la mesure réalisée, par exemple au moyen d'électrodes 30, le liquide est évacué via un canal de sortie 406 du corps hydraulique 4, il passe par le deuxième conduit 110, par l'électrovanne 6 (en sortant du socle 10a par l'entrée d'électrovanne 111, et réentrant par la sortie d'électrovanne 112), puis par le troisième conduit 113, et est enfin acheminé à un égout ou à l'entrée du système de purification de l'eau pour être recyclé, par le flexible de sortie 115.

Le socle 10a présente, d'un côté opposé à l'électrovanne 6, une butée 11, servant par exemple de détrompeur pour assembler le dispositif 1 et facilitant le positionnement du corps hydraulique 4 sur le socle 10a, notamment pour assurer que l'entrée 403 et la sortie 405 sont bien en vis-à-vis des orifices 108 et 109.

La fenêtre 2 est ici une simple plaque rectangulaire, transparente aux UV, réalisée par exemple en verre de quartz.

Selon des exemples de réalisation des figures 1 à 8 et 11 notamment, la chambre de mesure 400 formée dans le corps hydraulique 4 présente un fond formé par un substrat 3, de sorte que la fenêtre 2 et le substrat 3 sont situés de part et d'autre d'un corps hydraulique 4.

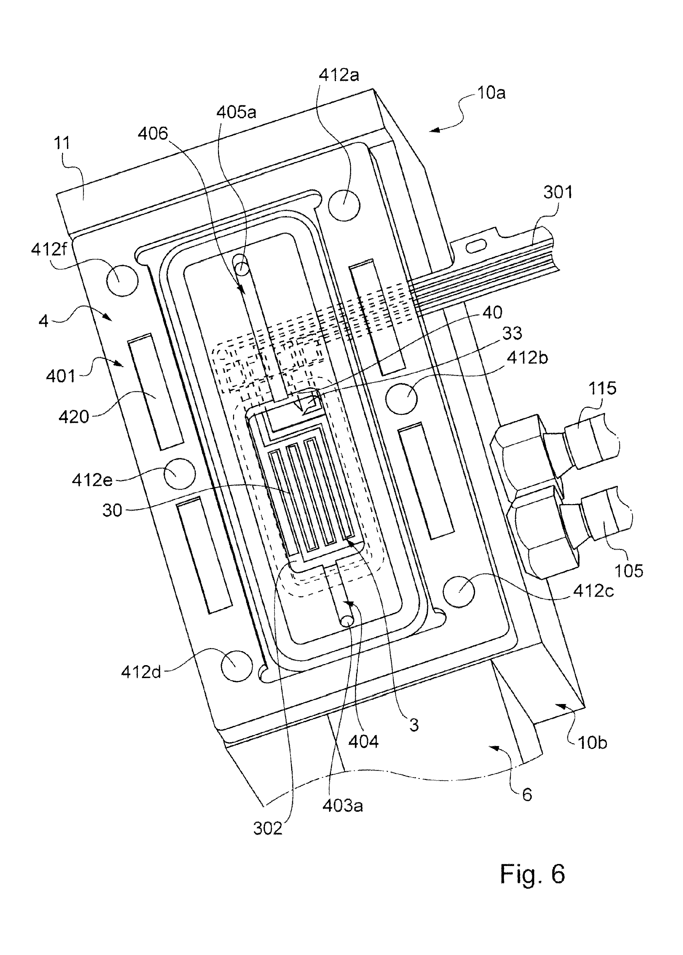

Selon un exemple de réalisation du substrat, par exemple représenté figures 2 et 6, deux électrodes 30 de mesure sont gravées sur une partie 302 d'une face 300 du substrat 3, dénommée ultérieure zone utile 302 destinée à être en contact avec le liquide présent dans la chambre de mesure 400 formée dans le corps hydraulique 4. Dans le présent exemple de réalisation, le substrat 3 présente une forme globalement rectangulaire et est par exemple également réalisé en verre de quartz. Outre les électrodes 30, le substrat 3 peut supporter, par exemple, un capteur de température 33 ou d'autres éléments électroniques nécessaires et d'usage dans ce type de dispositif, tel qu'un microcontrôleur par exemple, qui ne seront pas décrits plus en détail ici, comme cela est habituel à un homme du métier. De plus, un FPC (« Flexible Printed Circuit board ») 301 est brasé sur le substrat 3, en l'occurrence sur la face 300 du substrat 3, et de préférence sur une partie 303 distincte de la partie 302 présentant les électrodes 30. Tout contact électrique nécessaire est alors de préférence positionné sur la partie 303 de la face 300 ou au dos du substrat 3 afin d'être protégé de tout contact avec le liquide présent dans la chambre 400. Et de préférence, la zone utile 302 de la face 300 ne comprend que les électrodes 30, voire optionnellement le capteur de température 33, par exemple une thermistance. Dans le présent exemple de réalisation, les parties 302 et 303 sont juxtaposées selon une longueur du substrat 3, i.e. selon une direction longitudinale.

Il est plus ergonomique que la configuration du dispositif 1 permette d'avoir à la fois les flexibles d'arrivée de liquide 105 et de sortie 115, et le FPC d'un même côté. Et quel que soit le mode de réalisation du corps hydraulique 4, il est aussi plus pratique que l'entrée 403 soit positionnée vers l'électrovanne 6 et que la sortie 405 soit positionnée vers la lampe 5 en raison des poids de l'électrovanne 6 et de la lampe 5, surtout quand dispositif 1 est positionné à la verticale.

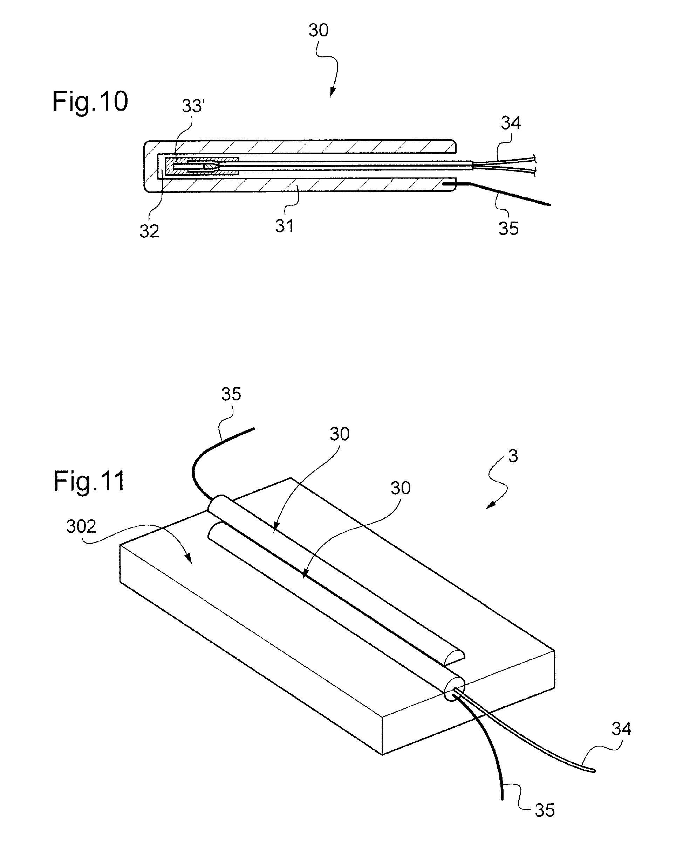

Dans l'exemple de réalisation de substrat 3 tel que représenté figure 11, le substrat 3 est alors principalement constitué d'une plaque de céramique ou de silicium où au moins une empreinte d'électrode est par exemple usinée. Le substrat 3 comprend ici deux électrodes 30 tête-bêche, dont au moins une comprend de préférence une thermistance 33', comme représenté figure 10. Ainsi le substrat 3 selon le mode de réalisation de la figure 11 est formé de la zone utile 302.

Une telle électrode 30 est par exemple composée d'un corps 31 conducteur électriquement, et thermiquement si l'électrode comprend une thermistance (par exemple en titane, optionnellement revêtues de platine ou d'or) auquel est relié un câble de connexion 35 pour récupérer la mesure, par exemple à un ordinateur (non représenté). Le cas échéant, une électrode 30 comprend une thermistance 33' immergée dans un liant conducteur thermique replissant un espace 32, et reliée aussi à un système de récupération de mesures par un câble de connexion 34 (par exemple à un ordinateur non représenté). L'électrode peut aussi comprendre tout autre câble nécessaire, par exemple de liaison terre ou autre.

Deux électrodes présentent par exemple une des configurations suivantes :

| Longueur d'une électrode (mm) | Diamètre (mm) | Distance entre deux électrodes (mm) |
|---|---|---|
| 18 | 1.2 | 1 |
| 18 | 1.2 | 0.5 |
| 28 | 1.2 | 1 |
| 28 | 1.2 | 0.5 |
| 18 | 2 | 1 |
| 18 | 2 | 0.5 |
| 28 | 2 | 0.5 |

Le corps hydraulique 4 comprend un circuit hydraulique permettant au fluide d'arriver dans la chambre de mesure 400, d'être analysé et puis évacué. Il présente une forme globalement parallélépipédique rectangle, et est de préférence réalisé en céramique à base d'au moins 16 % d'alumine, et de préférence vitrocéramique, en céramique injectée, ou en céramique usinable, ou par exemple en MACOR®, comme expliqué précédemment.

Le corps hydraulique 4 présente principalement une première surface 401, contre au moins une partie de laquelle est positionnée la fenêtre 2, et une deuxième surface 402 comprenant le fond de la chambre 400. Dans l'exemple de réalisation des figures 2 et 3 à 8 par exemple, le fond de la chambre 400 est formé par le substrat 3 qui est alors positionné contre une partie de la deuxième surface 402. Selon le présent exemple de réalisation, la deuxième surface 402 est opposée et parallèle à la première surface 401.

Selon cet exemple de réalisation, la chambre 400 est formée traversante dans le corps hydraulique 4 de sorte qu'elle débouche d'un côté sur la première surface 401 du corps hydraulique 4 et d'un autre côté sur la deuxième surface 402 du corps hydraulique 4. Elle est formée à coeur, au sein du corps hydraulique 4, mais est par exemple possiblement décentrée pour le passage d'un FPC, comme cela est détaillé ultérieurement.

Ainsi, le dispositif 1 présente un agencement tel que le substrat 3 est positionné contre une partie de la deuxième surface 402 du corps hydraulique 4, entre le corps hydraulique 4 et le socle 10a. On considère ainsi ici que la deuxième surface 402 est une surface inférieure du corps hydraulique 4. La fenêtre 2, quant à elle, est située entre le support 10b de la lampe 5 et le corps hydraulique 4, contre au moins une partie de la première surface 401 du corps hydraulique 4. On considère donc ici que la première surface 401 est une surface supérieure.

Bien entendu, les termes « inférieur », « supérieur », « première », « deuxième » sont arbitraires et ne sont utilisés ici que par souci de clarté en référence aux figures.

Plus précisément, la fenêtre 2 est positionnée de sorte à être à la fois en vis-à-vis de la chambre 400 et d'une ouverture (non visible sur les figures) du logement 100 de la lampe 5 permettant une irradiation focalisée sur un échantillon de liquide contenu dans la chambre 400. Et de façon analogue le substrat 3 est positionné contre une partie de la deuxième surface 402 tel que les électrodes 30, présentes sur la zone utile 302 soient en vis-à-vis de la chambre 400.

Ainsi, la fenêtre 2 transparente aux UV recouvre au moins une partie de la première surface 401 en fermant la chambre 400 du côté de la première surface 401, et le substrat 3 recouvre une partie de la deuxième surface 402 en fermant la chambre 400 du côté de la deuxième surface 402.

Pour la circulation du fluide, le corps hydraulique 4 comprend une entrée 403 pour alimenter la chambre 400 en liquide à mesurer et une sortie 405 pour évacuer le liquide une fois mesuré, l'entrée 403 et la sortie 405 étant situées hors de la partie de la deuxième surface 402 recouverte par le substrat 3. Ceci permet entre autre de pouvoir éviter tout usinage ou perçage du substrat 3 pour la circulation du fluide dans le dispositif 1. L'absence de trous dans la zone utile 302 du substrat 3 permet aussi de réduire la surface de cette zone utile, surface exposée aux rayons UV. Cette réduction est aussi facilitée par l'absence d'électrodes de photo-oxydation qui viennent parfois s'ajouter aux électrodes de mesure 30.

Dans le présent exemple de réalisation, l'entrée 403 et la sortie 405 sont formées dans la deuxième surface 402 hors d'une zone de positionnement du substrat 3, ou tel qu'il est possible de positionner le substrat 3 contre la deuxième surface 402 avec la zone utile 302 en vis-à-vis de la chambre 4 sans que le substrat 3 n'obstrue ni l'entrée 403 ni la sortie 405.

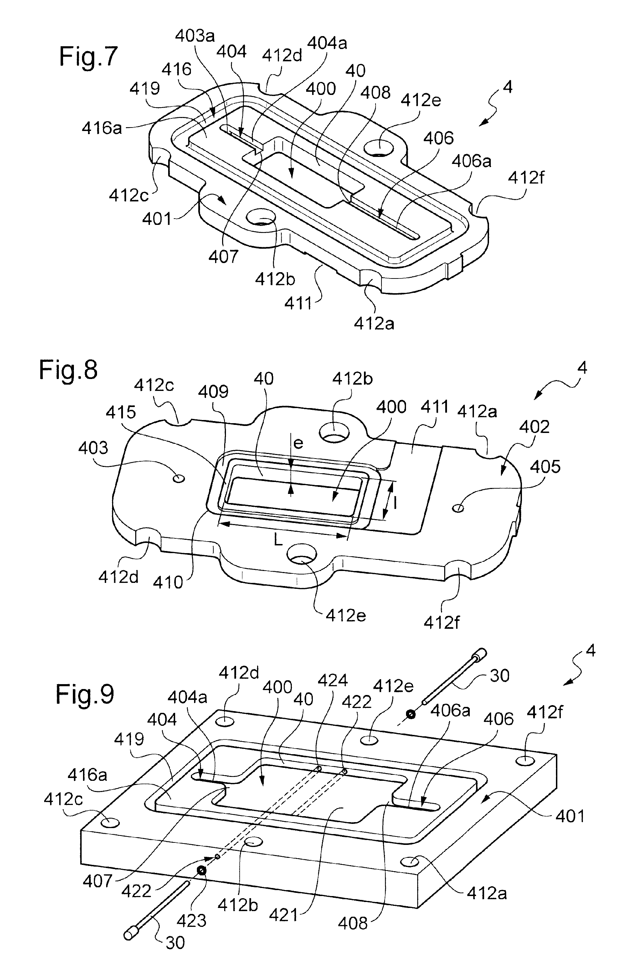

Dans le corps hydraulique 4, un canal d'entrée 404 est relié à l'entrée 403, et un canal de sortie 406 est relié à la sortie 405, le canal d'entrée 404 et le canal de sortie 406 débouchant tous deux dans la chambre 400, respectivement en des bouches 407 et 408 formées dans une paroi latérale 40 de la chambre 400, comme illustré figures 4, 7 et 9. On remarque, par exemple sur les figures 4 et 7, que le canal de sortie 406 est ici plus long que le canal d'entrée 404, ceci étant lié à l'excentration de la chambre 400 mentionnée précédemment. En effet, comme le montrent les figures 5 et 8, la deuxième surface 402 présente plusieurs empreintes 409, 411, qui servent notamment au positionnement du substrat 3 comprenant un FPC. Une empreinte 409, délimitée par un contour 410 entourant la chambre 400, permet d'y positionner le substrat 3 de sorte que la zone utile 302 comprenant les électrodes 30 est en vis-à-vis de la chambre 400. Elle est en outre creusée dans le corps hydraulique 4 et, ici, de taille ajustée à au moins une partie du substrat 3 pour y loger le substrat 3 de façon à ce que la zone utile 302 de la face 300 soit positionnée le plus centrée possible par rapport à la chambre 400. Le contour 410 pourrait toutefois présenter n'importe quelle forme tant qu'il permet de positionner le substrat 3 avec sa zone utile 302 en vis-à-vis de la chambre 400. Une autre empreinte est formée d'une creusure 411 recoupant l'empreinte 409 pour faire passer le FPC 301 brasé sur le substrat 3. Dans le présent exemple de réalisation, le FPC 301 étant brasé au substrat 3 par la partie 303 de la face 300, et du fait que la zone utile 302 de la face 300 comprenant les électrodes est en vis-à-vis de la chambre 400, il est préférable que la creusure 411 ait une profondeur supérieure à l'empreinte 409 destinée au substrat. De par cette disposition du substrat 3, le canal de sortie 406 est plus long que le canal d'entrée 404 de sorte à relier la chambre 400 à la sortie 405, en enjambant la partie 303 de la face 300 du substrat 3. Bien sûr, d'autres configurations sont envisageables sans sortir du cadre de la présente invention, comme par exemple renverser cette dissymétrie de sorte que la partie 303 soit située vers l'entrée 403 par exemple, ou encore que la partie 303 ne soit non plus située dans un prolongement longitudinal de la zone utile 302 mais soit par exemple juxtaposée selon une largeur du substrat. Toutefois, la présente configuration présente par exemple l'avantage de permettre de faire passer le FPC 301 vers l'extérieur du dispositif 1, sans gêner l'assemblage ou l'étanchéité de l'ensemble.

Ainsi, dans le présent exemple de réalisation, la creusure 411 est formée entre deux trous traversant 412a et 412b du corps hydraulique 4 à travers lesquels passent respectivement des éléments de fixation 101 a et 101 b, permettant de serrer ensemble les deux parties 10a et 10b du boitier 10 du dispositif 1.

Dans cet exemple, six éléments de fixation 101 (a à f), qui sont par exemple des vis, sont prévus, mais leur nombre est bien évidemment variable. Ils traversent chacun un trou 102 prévu dans le support 10b, un des trous 412 (a à f) du corps hydraulique 4, et viennent se fixer dans des trous 103 du socle 10a, par exemple par vissage. La fenêtre 2, le corps hydraulique 4 et le substrat 3 sont ainsi légèrement comprimés entre le support 10b et le socle 10a pour garantir une étanchéité, qui est optionnellement renforcée par différents joints.

Dans l'exemple de réalisation des figures 7 et 8, le corps hydraulique 4 présente une forme permettant notamment de minimiser la quantité de matière nécessaire à sa réalisation. Seuls les trous 412b et 412e sont entiers, les autres ayant été tronqués.

Pour renforcer l'étanchéité, par exemple, le corps hydraulique 4 présente optionnellement un renfoncement 413 autour de l'entrée 403 et un renfoncement 414 autour de la sortie 405 qui sont par exemple destinés à recevoir chacun un joint torique 104a, 104b (visibles par exemple figure 2) comme le montre l'exemple de réalisation de la figure 5, alors que celui de la figure 8 en est dépourvu.

L'empreinte 409, destinée à recevoir au moins une partie du substrat 3, comprend aussi par exemple une rainure 415, entourant la chambre 400, pour y recevoir un joint (non représenté) renforçant l'étanchéité et notamment l'isolement de la zone utile 302 de la partie 303 du substrat 3 par exemple.

De façon analogue, en référence au mode de réalisation de la figure 4, la première surface 401 comprend une empreinte 416, délimitée par un contour 417 entourant la chambre 400, destinée à recevoir la fenêtre 2. Ici, l'empreinte 416 est creusée et présente une forme globalement rectangulaire. Elle comprend optionnellement près de chacun de ses coins un renfoncement 418 formé par un décrochement, ici de forme arrondie, dans le contour 417. Les renfoncements 418 permettent par exemple de faire passer un outil ou un doigt pour déloger la fenêtre 2, par exemple pour nettoyage du dispositif 1. Ainsi, lorsque la fenêtre 2 est positionnée dans son empreinte 416, le contour 417 est en contact discontinu avec la fenêtre 2. De même que précédemment, l'empreinte 416 et son contour 417 pourraient présenter n'importe quelle forme tant que la fenêtre en positionnée en vis-à-vis de la chambre 400 et permet de la clore. La première surface 401 présente en outre une rainure 419 pour y recevoir un joint (non représenté) pour renforcer l'étanchéité entre la chambre 400 et la fenêtre 2 lorsque le dispositif 1 est assemblé.

Dans l'exemple de réalisation de la figure 7, l'empreinte 416 est formée par la rainure 419.

Toutefois, dans un mode de réalisation où la rainure 419 et l'empreinte 416 sont distinctes, il est préférable que la rainure 419 soit positionnée entre un contour 417 de l'empreinte 416 et la chambre 400.

Dans ces exemples de réalisation, le contour 417 de l'empreinte 416 voire la rainure 419 si elle existe entoure non seulement la chambre 400, mais aussi au moins une portion du canal d'entrée 404 et du canal de sortie 406 de sorte qu'une embouchure 403a reliée à l'entrée 403 par une portion du canal d'entrée 404 et une embouchure 405a reliée à la sortie 405 par une portion du canal de sortie 406 sont comprises dans la zone délimitée par la rainure 419 si elle existe ou le contour 417, i.e. celui des deux le plus proche de la chambre 400.

Les embouchures 403a et 405a ne sont pas nécessairement situées au droit de l'entrée 403 et de la sortie 405. Elles peuvent être décalées, par exemple recentrées, rapprochées l'une de l'autre par rapport à l'entrée 403 et à la sortie 405. Ceci implique qu'en soi, l'entrée 403 et la sortie 405 peuvent être situées hors du contour 417 et/ou de la rainure 419 si ceci était tracé à l'identique sur la deuxième surface 402 du corps hydraulique 4. Le canal d'entrée 404 et le canal de sortie 406 présentent alors par exemple un angle obtus, c'est-à-dire supérieur à un angle droit comme illustré dans le présent exemple de réalisation. Un angle obtus est en outre préférable à un angle aigu pour éviter de perturber l'écoulement.

Afin de favoriser l'écoulement en évitant au mieux des formations de bulles ou de turbulences, il est préférable que le canal d'entrée 404 et le canal de sortie 406 débouchent dans la chambre 400 de façon parallèle au fond de la chambre 400, ici le substrat 3, et/ou de la fenêtre 2. Le canal d'entrée 404 présente une portion 404a comprise entre l'embouchure 403a et la bouche 407 parallèle au fond de la chambre 400, et en outre ici rectiligne, et le canal de sortie 406 présente une portion 406a comprise entre l'embouchure 405a et la bouche 408 parallèle au fond de la chambre 400, et aussi ici rectiligne. De plus, les portions 404a et 406a sont ici dans le prolongement l'une de l'autre et en vis-à-vis.

Pour faciliter la réalisation du corps hydraulique, et/ou son entretien, les portions 404a et 406a sont ouvertes, i.e. formées par des gorges creusées dans la première surface 401, dans l'empreinte 416, de sorte que l'écoulement est dirigé et les canaux 404 et 406 clos jusqu'aux bouches 407 et 408 par la fenêtre 2, tangente, et en contact étanche avec au moins une partie 416a entourant directement la chambre 400 et les portions 404a et 406a des canaux 404 et 406. Une telle conception des canaux d'entrée 404 et de sortie 406 permet ainsi un nettoyage facile de l'ensemble du circuit hydraulique du corps hydraulique 4 puisque toutes les parties du circuit sont visibles, et accessibles. De plus, un coude formé dans les canaux d'entrée 404 et de sortie 406 est de préférence arrondi pour limiter toute formation de turbulences dans l'écoulement.

Le corps hydraulique 4 comprend enfin, optionnellement différentes autres empreintes, par exemple les empreintes rectangulaires creusées 420 (par exemple sur le mode de réalisation des figures 4 et 5), permettant par exemple d'ajouter différents joints pour l'étanchéité si nécessaire, pour pouvant coopérer avec des formes complémentaires du boitier 10 pour la réalisation de l'assemblage du dispositif 1.

Différentes formes de joints peuvent être réalisées, comme par exemple des joints à lèvre pour épouser la forme du corps hydraulique et éviter un volume mort d'eau situé au niveau de la partie 416a.

Les matériaux des joints étant sélectionnés pour avoir peu de résidus organiques rejetés lors d'une irradiation aux rayons UV et au contact avec de l'eau pure ou ultra-pure. Ces matériaux pouvant être à base de polymère fluorocarbone par exemple (PTFE, PEEK, Viton®, nitrile ... ). Ces joints peuvent être obtenus classiquement par moulage, presse, par exemple.

Le mode de réalisation de la figure 9 présente un corps hydraulique 4 dans lequel la chambre 400 comprend un fond 421 faisant ici partie intégrante du corps hydraulique 4. Il pourrait aussi être une plaque indépendante, optionnellement de la même matière, par exemple.

La chambre 400 est ici centrée selon la largeur et la longueur par rapport au corps hydraulique 4 du fait de l'absence de FPC sur un substrat, mais sa profondeur est inférieure à celle du corps hydraulique 4 de sorte que le fond 421 est tangent aux portions 404a et 406a des canaux d'entrée 404 et de sortie 406. Ainsi les portions 404a et 406a sont à la fois tangentes au fond 421 et à la partie 416a, ils présentent une hauteur identique à l'épaisseur de la chambre 400.

De plus, les bouches 407 et 408 présentent une forme évasée, d'entonnoir, afin de minimiser encore les perturbations de l'écoulement.

Les capteurs sont ici réalisés par deux électrodes 30 tête-bêche, dont au moins une comprend de préférence une thermistance, insérées de part et d'autre du corps hydraulique 4 via des canaux 422 (optionnellement pourvu d'un joint 423). Les électrodes 30 sont par exemple du type de celles décrites précédemment en référence à la figure 10. Optionnellement, un renfoncement 424 permet d'accueillir une extrémité de l'électrode 30 pour éviter qu'elle ne soit en porte-à-faux dans son canal 422. De plus, les électrodes 30 sont par exemple positionnées transversalement à l'écoulement, voire orthogonalement. Cela permet de mieux garantir leur totale immersion quelle que soit l'orientation de la chambre de mesure 400 (parfois positionnée verticalement).

Les différentes caractéristiques présentées en référence aux trois modes de réalisation précédemment détaillés sont bien entendu combinables selon les besoins, à l'appréciation de l'homme du métier.

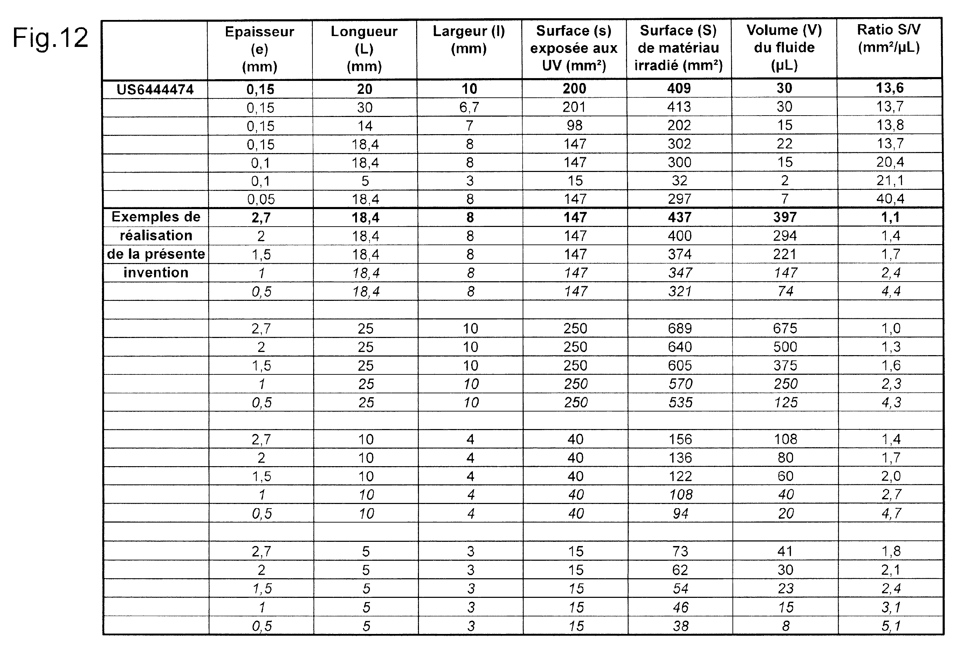

La figure 12 permet de présenter différents modes de réalisation des dimensions de la chambre 400, et comparer les ratios (SN) correspondant avec ceux des dispositifs présentés dans le document US 6 444 474.

La chambre 400, de forme globalement parallélépipédique rectangle, présente, par exemple en référence à la première ligne se rapportant à la présente invention, une longueur (L) de 18,4 mm, une largeur (I) de 8 mm et une épaisseur (e) de 2,7 mm, soit un volume (V) d'environ 397 μL. La surface totale irradiée (S) est déterminée par la formule suivante : 2 * surface exposée aux rayons (s = L * I) + surface latérale (2*(L+I)*e).

Dans le cas présent, la surface totale irradiée est de :

$$2*(18,4*8) + 2*(18,4+8)*2,7 = 436,96 \text{ mm}^2.$$

Ainsi, les dimensions des éléments sont telles que le ratio (SN) est de 436,96/397 = 1,1 mm$^2$/μL.

Ce tableau montre ainsi, pour différentes dimensions de chambre, l'influence de l'épaisseur et de la surface active sur le ratio (S/V). Une épaisseur inférieure à 150 μm et un volume d'eau inférieur à 30 μL couplés à des électrodes de photo-catalyse, comme indiqués dans le brevet US 6 444 474, permettent une photo-oxydation très rapide du fluide mais donne un ratio (S/V) supérieur à 13 mm$^2$/μL quelle que soit la géométrie de la chambre, le générateur d'extractibles empêchant alors les mesures de faible TOC. L'arrivée et la sortie du fluide dans la chambre de mesure parallèlement au rayonnement UV ainsi que les électrodes de photo-catalyse nécessitent de la place, empêchant la miniaturisation de la chambre, voire de la cellule,

Du fait que la présente invention permet notamment de minimiser la surface (S) de matériaux irradiés tout en maximisant le volume (V), il est ainsi possible de réduire les ratios (S/V).

Toutefois, le volume (V) est borné par l'épaisseur (e) de la chambre 400.

En effet, comme le montre la figure 13, l'intensité relative des rayons UV décroit fortement en fonction de la profondeur dans le fluide (ici de l'eau ultra-pure). Ainsi au-delà d'une certaine épaisseur de l'échantillon, et donc de la chambre 400, le rayonnement s'affaiblit de sorte que l'irradiation de l'échantillon est moins efficace. Par conséquent, il est préférable que l'épaisseur de la chambre reste inférieure ou égale à 5 mm pour que l'ensemble de l'échantillon de fluide soit irradié à au moins 60% des rayons UV appliqués.

La figure 14, à comparer à la courbe « triangle » de la figure 5 du document US 6 444 474, permet ainsi d'apprécier les avantages en termes de lessivage apportés par la présente invention dans le cas où le dispositif est réalisé en MACOR®. La figure 14 montre l'évolution de la conductivité (en μS/cm) en fonction du temps (en secondes). La courbe avec les « carrés » représente l'évolution de la conductivité dans une eau pure comprenant initialement 10 ppb de composés organiques, et la courbe avec les « triangles » représente l'évolution de la conductivité dans de l'eau dé-ionisée (faisant office de témoin pour apprécier le lessivage du dispositif).

Théoriquement, la conductivité après oxydation d'une eau pure, initialement à 0,86 μS/cm comprenant 10 ppb de composés organiques, atteint 0,8995 μS/cm, soit une augmentation de 4 %.

Comme le montre la courbe « triangle » de la figure 5 du document US 6 444 474, le lessivage produit une variation de conductivité de 5,2 μS/cm/min, ce qui induirait une erreur constante de 520 %. Une variation de 2,6 % n'est donc pas détectable.

Comme le montre la figure 14, un dispositif selon l'invention permet de mesurer des teneurs mille fois plus petites. Le lessivage selon la courbe de « triangle » montre une évolution de la conductivité due au lessivage de 0,0055

$\mu$S/cm/min, ce qui permet ainsi de réaliser des analyses au niveau de quelques ppb.

Bien sûr, la présente invention ne se limite pas à la description précédente, mais s'étend à toute variante dans le cadre des revendications ci-après.

## Revendications

1. Dispositif (1) de mesure de conductivité d'un liquide, comprenant une chambre de mesure (400) pour contenir un volume d'échantillonnage à irradier par rayons UV, une fenêtre (2) transparente aux UV étant située entre la chambre de mesure (400) et une source de rayons UV (5) en fermant hermétiquement un premier côté de la chambre de mesure (400), **caractérisé en ce que** la chambre (400) est formée dans un corps hydraulique (4), la chambre de mesure (400) débouchant au moins du premier côté sur une première surface (401) du corps hydraulique (4), la fenêtre (2) transparente aux UV recouvrant au moins une partie de la première surface (401) en fermant la chambre de mesure (400) hermétiquement du côté de la première surface (401), et **en ce que** le corps hydraulique (4) comprend un canal d'entrée (404) pour alimenter la chambre de mesure (400) en liquide à mesurer et un canal de sortie (406) pour évacuer le liquide mesuré de la chambre de mesure (400), le canal d'entrée (404) et le canal de sortie (406) débouchant de part et d'autre hors d'une surface exposée aux rayons UV, de sorte que seul le volume d'échantillonnage contenu dans la chambre de mesure (400) est irradié.

2. Dispositif selon la revendication 1, **caractérisé en ce que** le corps hydraulique (4) comprend une entrée (403) à laquelle est relié d'une part le canal d'entrée (404), et une sortie (405) à laquelle est relié d'une part le canal de sortie (406), le canal d'entrée (404) et le canal de sortie (406) débouchant d'autre part dans la chambre (400) par une paroi latérale (40, 70, 80) délimitant latéralement la chambre de mesure (400).

3. Dispositif selon l'une quelconque des revendications 1 ou 2, **caractérisé en ce que** la chambre de mesure (400) est fermée d'un côté d'une deuxième surface (402) du corps hydraulique (4) par un fond (81) faisant partie intégrante du corps hydraulique (4).

4. Dispositif selon l'une quelconque des revendications 1 ou 2, **caractérisé en ce que** la chambre de mesure (400) est fermée hermétiquement d'un côté d'une deuxième surface (402) du corps hydraulique (4) par un substrat (3) comportant au moins deux électrodes (30) de mesure de conductivité, le substrat (3) étant appliqué contre au moins une partie de la deuxième surface (402) du corps hydraulique (4) tel que les électrodes (30) sont en vis-à-vis de la chambre de mesure (400).

5. Dispositif (1) selon l'une quelconque des revendications 1 à 4, **caractérisé en ce qu'**une portion (404a) du canal d'entrée (404) et une portion (406a) du canal de sortie (406) débouchent dans la chambre (400) parallèlement à la fenêtre (2) et sont formées chacune dans le corps hydraulique (4) par une gorge creusée dans la partie de la première surface (401) recouverte par la fenêtre (2) transparente aux UV.

6. Dispositif (1) selon la revendication 4, et l'une quelconque des revendications 1, 2 ou 5, **caractérisé en ce que** la deuxième surface (402) du corps hydraulique (4) comprend une empreinte (409) délimitée par un contour (410) entourant la chambre (400) pour positionner le substrat (3) comprenant des électrodes (30) de sorte que les électrodes (30) sont en vis-à-vis de la chambre de mesure (400), et l'empreinte (409) étant creusée dans le corps hydraulique (4) et de taille ajustée au substrat (3) pour l'y loger.

7. Dispositif (1) selon l'une quelconque des revendications 1 à 6, **caractérisé en ce que** la première surface (401) du corps hydraulique (4) comprend une empreinte (416) délimitée par un contour (417) entourant la chambre de mesure (400), pour positionner la fenêtre (2) transparente aux UV, et l'empreinte (416) étant creusée dans le corps hydraulique (4) et de taille ajustée à la fenêtre (2) pour l'y loger.

8. Dispositif (1) selon l'une quelconque des revendications 1 à 7, **caractérisé en ce qu'**il comprend une thermistance logée au sein d'une électrode (30) de mesure.

9. Dispositif (1) selon les revendications 3 et 8 et l'une quelconque des revendications 1, 2, 5, et 7, **caractérisé en ce que** l'électrode (30) de mesure comprenant la thermistance est introduite dans la chambre de mesure (400) via un orifice formé latéralement dans le corps hydraulique (4).

10. Dispositif (1) selon l'une quelconque des revendications 1 à 9, **caractérisé en ce que** le corps hydraulique (4) est en céramique comprenant au moins 16 wt.% d'alumine.

**11.** Dispositif (1) selon la revendication 10, **caractérisé en ce que** la céramique du corps hydraulique (4) est une vitrocéramique usinable.

**12.** Dispositif (1) selon la revendication 10 ou 11, **caractérisé en ce que** le corps hydraulique (4) est en MACOR®.

**13.** Dispositif (1) selon la revendication 10 ou 11, **caractérisé en ce que** le corps hydraulique est composé d'une céramique comprenant au moins 99 % d'alumine.

**14.** Dispositif (1) selon l'une quelconque des revendications 1 à 13, **caractérisé en ce que** la chambre de mesure (400) présente une épaisseur (e) comprise entre 0,5 mm et 4 mm, un volume (V) supérieur ou égal à 400 $\mu$L, et une surface de matériau irradié (S) inférieure ou égale à 600 mm$^2$.

**15.** Utilisation d'un dispositif (1) selon l'une quelconque des revendications 1 à 14, **caractérisée en ce que** le dispositif (1) est positionné de sorte qu'un écoulement de fluide dans la chambre de mesure (400) soit vertical et ascendant, le canal d'entrée (404) débouchant dans la chambre (400) sous le canal de sortie (406).

**16.** Système de purification d'eau, **caractérisé en ce qu'**il comprend un dispositif (1) selon l'une quelconque des revendications 1 à 14, le dispositif (1) étant fixé sur une carte électronique tel que le canal d'entrée (404) débouche dans la chambre (400) sous le canal de sortie (406) de sorte qu'un écoulement de fluide dans la chambre de mesure (400) soit vertical et ascendant, le canal d'entrée (404) et le canal de sortie (406) présentant au moins chacun une portion (404a, 406a) dans le prolongement l'une de l'autre, les portions (404a, 406a) débouchant dans la chambre en vis-à-vis.

Fig.1

Fig.2

Fig.3

Fig.4

Fig.5

Fig. 6

Fig.7

Fig.8

Fig.9

Fig.10

Fig.11

Fig.12

EP 2 803 996 A1

|  | Epaisseur (e) (mm) | Longueur (L) (mm) | Largeur (l) (mm) | Surface (s) exposée aux UV (mm²) | Surface (S) de matériau irradié (mm²) | Volume (V) du fluide (µL) | Ratio S/V (mm²/µL) |
|---|---|---|---|---|---|---|---|
| US6444474 | 0,15 | 20 | 10 | 200 | 409 | 30 | 13,6 |
|  | 0,15 | 30 | 6,7 | 201 | 413 | 30 | 13,7 |
|  | 0,15 | 14 | 7 | 98 | 202 | 15 | 13,8 |
|  | 0,15 | 18,4 | 8 | 147 | 302 | 22 | 13,7 |
|  | 0,1 | 18,4 | 8 | 147 | 300 | 15 | 20,4 |
|  | 0,1 | 5 | 3 | 15 | 32 | 2 | 21,1 |
|  | 0,05 | 18,4 | 8 | 147 | 297 | 7 | 40,4 |
| Exemples de | 2,7 | 18,4 | 8 | 147 | 437 | 397 | 1,1 |
| réalisation | 2 | 18,4 | 8 | 147 | 400 | 294 | 1,4 |
| de la présente | 1,5 | 18,4 | 8 | 147 | 374 | 221 | 1,7 |
| invention | 1 | 18,4 | 8 | 147 | 347 | 147 | 2,4 |
|  | 0,5 | 18,4 | 8 | 147 | 321 | 74 | 4,4 |
|  |  |  |  |  |  |  |  |
|  | 2,7 | 25 | 10 | 250 | 689 | 675 | 1,0 |
|  | 2 | 25 | 10 | 250 | 640 | 500 | 1,3 |
|  | 1,5 | 25 | 10 | 250 | 605 | 375 | 1,6 |
|  | 1 | 25 | 10 | 250 | 570 | 250 | 2,3 |
|  | 0,5 | 25 | 10 | 250 | 535 | 125 | 4,3 |
|  |  |  |  |  |  |  |  |
|  | 2,7 | 10 | 4 | 40 | 156 | 108 | 1,4 |
|  | 2 | 10 | 4 | 40 | 136 | 80 | 1,7 |
|  | 1,5 | 10 | 4 | 40 | 122 | 60 | 2,0 |
|  | 1 | 10 | 4 | 40 | 108 | 40 | 2,7 |
|  | 0,5 | 10 | 4 | 40 | 94 | 20 | 4,7 |
|  |  |  |  |  |  |  |  |
|  | 2,7 | 5 | 3 | 15 | 73 | 41 | 1,8 |
|  | 2 | 5 | 3 | 15 | 62 | 30 | 2,1 |
|  | 1,5 | 5 | 3 | 15 | 54 | 23 | 2,4 |
|  | 1 | 5 | 3 | 15 | 46 | 15 | 3,1 |
|  | 0,5 | 5 | 3 | 15 | 38 | 8 | 5,1 |

# Fig.13

# Fig.14

Europäisches
Patentamt

European
Patent Office

Office européen
des brevets

**RAPPORT DE RECHERCHE EUROPEENNE**

Numéro de la demande

EP 13 29 0107

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 5 275 957 A (BLADES FREDERICK K [US] ET AL) 4 janvier 1994 (1994-01-04) * abrégé * * colonne 12 - colonne 13; figure 11 * * colonne 35 - colonne 36; figure 21 * ----- | 1-16 | INV. G01N33/18 |
| X,D | US 6 444 474 B1 (THOMAS ROSS C [US] ET AL) 3 septembre 2002 (2002-09-03) * colonne 8; figure 1a * ----- | 1 | |

DOMAINES TECHNIQUES RECHERCHES (IPC)

G01N

Le présent rapport a été établi pour toutes les revendications

| Lieu de la recherche | Date d'achèvement de la recherche | Examinateur |
|---|---|---|
| La Haye | 6 septembre 2013 | Steinmetz, Johannes |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons

............................................................................

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 13 29 0107

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

06-09-2013

| Document brevet cité au rapport de recherche | Date de publication | Membre(s) de la famille de brevet(s) | Date de publication |
|---|---|---|---|
| US 5275957 A | 04-01-1994 | AUCUN | |
| US 6444474 B1 | 03-09-2002 | AUCUN | |

EPO FORM P0460

Pour tout renseignement concernant cette annexe : voir Journal Officiel de l'Office européen des brevets, No.12/82

## RÉFÉRENCES CITÉES DANS LA DESCRIPTION

**Documents brevets cités dans la description**

- EP 1927849 A

- US 6444474 B